# EUROPEAN PATENT SPECIFICATION

(11) **EP 1 750 717 B1**
(45) Date of publication and mention of the grant of the patent: **19.07.2017**
(21) Application number: 05702468.9
(22) Date of filing: 10.02.2005
(51) Int. Cl.: A61K 9/00, A61K 31/366, A61K 31/55, A61K 31/7052, A61K 47/10, A61K 47/38, A61K 31/41, A61K 31/522, A61K 9/20

(54) **CONTROLLED RELEASE PHARMACEUTICAL COMPOSITIONS WITH IMPROVED BIOAVAILABILITY**
PHARMAZEUTISCHE ZUSAMMENSETZUNGEN MIT KONTROLLIERTER FREISETZUNG UND VERBESSERTER BIOVERFÜGBARKEIT
COMPOSITIONS PHARMACEUTIQUES A LIBERATION CONTROLEE PRESENTANT UNE MEILLEURE BIODISPONIBILITE

(30) Priority: 11.02.2004 AU 2004900661
(43) Date of publication of application: 14.02.2007
(73) Proprietor: Rubicon Research Private Limited, Mumbai 400 078 Maharashtra (IN)
(72) Inventor: PILGAONKAR, Pratibha, Mumbai 400 081 (IN); BAGDE; Pradnya M., Wagle Estate, Thane (W) 400-604 (IN); RUSTOMJEE, Maharukh T., Mumbai 400 007 (IN); GANDHI, Anilkumar S., Road, Borivali (W.), Mumbai 400092 (IN)
(74) Representative: HGF Limited
(86) International application number: PCT/IB2005/000330
(87) International publication number: WO 2005/079752

(56) References cited:
- EP-A- 1 269 996
- WO-A-01/22791
- WO-A-02/28373
- WO-A-02/092078
- US-A- 5 085 865
- US-A1- 2003 232 081

## Description

### Field of invention

The present invention relates to controlled release oral pharmaceutical compositions with improved bioavailability having at least one active pharmaceutical ingredient of low bioavailability. In particular, the present invention relates to a controlled release pharmaceutical composition where its bioavailability is improved by solubilizing the active ingredient using a solubilizer and incorporating it in a gastro-retentive system.

### Background of the Invention

Certain pharmaceutical active agents are not easily absorbed from the gastrointestinal tract or do not dissolve readily in the medium of gastrointestinal tract. For such pharmaceuticals, bioavailability is usually low and unfortunately creates a need for frequent dosing of a large amount of the pharmaceutical in order to provide and maintain therapeutic levels. The need for frequent dosing presents patient compliance problems and the need for large amount of active ingredient may result in increased toxicity.

For pharmaceuticals whose bioavailability is limited by solubility or dissolution rate various attempts have been made in the prior art to improve solubility or dissolution rate. In US Patent No. 4,973,469 ('469 patent) a process of preparing a controlled release formulation by preparing an adsorbate of drug and inactive substance on to copovidone (copolymer of N-vinyl-2-pyrrolidone and vinyl acetate) is described. According to the '469 patent, a water-insoluble inactive substance serves to impede the rate of dissolution whereas a water-soluble substance would result in leaching of drug adsorbed on the crosslinked polymer. The '469 patent however, does not disclose any method or composition to increase the solubility of the drug which is very important for increasing bioavailability of poorly soluble drugs.

In US Patent No. 6,699,503 ('503 patent) a hydrogel type sustained release preparation is disclosed comprising at least one drug, an additive which insures penetration of water into the core of the preparation and a hydrogel forming polymer. Due to the presence of a hygroscopic agent that pulls water into the preparation a gel is formed and the release of the drug is apparently enhanced. Unfortunately, the pulling of water into the system using hygroscopic agent does not necessarily ensure increase in dissolution rate or release of poorly soluble drug.

In US Patent No. 5,945,125 ('125 patent), a controlled release tablet formulation containing a pharmaceutical agent and a water-swellable polymer is disclosed such that a zero order release rate is achieved. While this disclosure aims to achieve a zero order controlled release formulation, it does not attempt to increase bioavailability of the active agent.

All the above prior approaches are primarily aimed at process for enhancing or retarding the release of an active pharmaceutical ingredient or a process of achieving a specific dissolution profile. Unfortunately, mere increases in dissolution rate may not ensure improved bioavailability as solubility of the drug is not altered. These prior approaches do not result in the reduction in dose of a drug and associated benefits such as reduction in side effects, patient compliance etc.

In US Patent No. 5,736,161 ('161 patent) methods and composition are disclosed for improving the oral absorption of drug by means of encapsulation in millispheres of gellable hydrocollids covered with positively charged polysaccharide. Unfortunately, the processes involved in the preparation of millispheres and encapsulation of drugs therein are tedious, expensive and difficult to produce on a commercial scale.

In WO03000294 a pharmaceutical composition with a solid dispersion of a low solubility drug and a matrix forming agent combined with a polymer is disclosed. A major portion of the drug is in the amorphous form. The composition apparently provides improved solubility, bioavailability and stability of the active ingredient. Also a method of achieving controlled release of the active in amorphous form is described. The WO03000294 disclosure is not suitable for drugs having a narrow window of absorption as only part of the drug will be released near the absorption window and remaining drug would be lost unabsorbed.

In US Patent No. 6,107,276 ('276 patent) pharmaceutical compositions of slightly soluble drugs are described. The composition includes a surface active agent and an oil in which the drug is dispersed which is adsorbed on to crospovidone (crosslinked polyvinyl pyrrolidone). This apparently results in improved dissolution and consequently improved bioavailability. The formulation may also be adopted for controlled release of the solubilized active. However, this approach is not suitable for drugs which are only absorbed in the upper segments of gastrointestinal tract.

As seen from the prior approaches, though various methods such as complexation, change in crystalline form of drug or preparation of micro-emulsion etc are made to increase the solubility of the low solubility drug. Unfortunately, many of these processes encounter difficulties during commercial scale manufacture. Some of these concepts have been utilized further to formulate controlled release oral dosage forms. A controlled release of a solubilized drug will only result in substantial improvement of bioavailability for drugs that are absorbed throughout the gastrointestinal tract. These prior approaches have proven not to be useful for drugs having a narrow window of absorption in the gastrointestinal tract, which demands the release of solubilized drug at or near the site of absorption in order to achieve improved bioavailability.

US Patent No. 5,780,057 ('507 patent) describes a pharmaceutical dosage form for oral administration comprising of 2 or 3 layer tablets where at least one layer can rapidly swell by contact with biological and/ or aqueous fluids, said swelling resulting in a considerable increase in the tablet volume resulting in gastric retention. The '507 patent discloses a dosage form allowing a slow release of the active ingredient to the stomach and/or the first tract of the intestines. This multilayered system is useful only for pharmaceutical active ingredients having high aqueous solubility. For pharmaceuticals having low solubility, release from such a system would be prolonged to an extent that a sizeable amount of drug would remain unreleased.

US Patent No. 6,340,475 ('475 patent), describes a water soluble drug formulated as unit dosage form by incorporating it into polymeric matrices comprised of hydrophilic polymer that swell upon imbibing water, to a size that is large enough to promote retention of the dosage form in the stomach during the fed mode. While it is helpful that the delivery system be adapted to remain in the stomach for a prolonged period, it is important that the system deliver active agent in a controlled manner. Unfortunately, these systems would not be suitable for low solubility pharmaceuticals as the release of these would be dramatically retarded from such systems.

US Patent No. 6,120,803 describes compositions where the dosage form of the active agent is a polymer matrix that swells upon contact with fluid of stomach. A portion of the polymer matrix is surrounded by a band of insoluble material that prevents the concerned portion of polymer matrix from swelling and provides a segment of the dosage form that is of sufficient rigidity to with stand the environment of the stomach and delay expulsion of the dosage form from the stomach until substantially all of the active agent has been dispersed. This disclosure describes a special kind of gastroretentive system with a polymer band of insoluble material. Application of such a band on the tablets needs special equipment and is difficult to produce on a commercial scale.

US Patent No. 6,022,562 discloses microcapsules for oral administration of medicinal and nutritional active principles which are smaller than 1000 µm and which are claimed to remain in the small intestine for a longer time (at least 5 hrs) allowing for the release and absorption of the active principles. Although these microcapsules are claimed to remain in the intestine for long duration, they would be emptied rather rapidly from stomach and upper gastrointestinal tract, the main site of drug absorption..

It is thus evident that many prior attempts have been made to formulate gastro-retentive compositions utilizing various techniques like increasing the size of the tablets after ingestion, or inclusion of a non-swellable band, or a bio-adhesive composition, or preparation of microspheres. In these systems the active agent is released by diffusion or a combination of diffusion and erosion. The majority of the prior approaches are with water-soluble active agents where due to high solubility, the drug is released by diffusion over a desired length of time.

It is a significant challenge to develop a gastroretentive system for poorly soluble drugs where release of drug through diffusion is restricted by solubility of the drug. Poor solubility may result in prolongation of release beyond the retention time and loss of unabsorbed drug. Some of the prior approaches describe eroding matrices, however, it would be still difficult to achieve a balance between the desired release of the drug through erosion and gastroretention as they are mutually antagonistic.

It has been surprisingly found that when a solubilized drug is incorporated in gastroretentive system the desired delicate balance of release and retention could be achieved. The present invention describes the compositions of sparingly soluble drugs having improved instantaneous solubility. These solubilized drugs when formulated in a controlled release swelling matrix, achieve more than 80% drug release in 12 hrs in dissolution studies; this was not possible to attain when such drugs were available in either only solubilized compositions or only controlled release compositions as such. Increase in solubility and release of drug near absorption site ensures better absorption of the drug resulting in increased bioavailability. Increased bioavailability coupled with extended released would mean reduction in dose, dosage frequency, improved patient compliance and more importantly enhanced therapeutic benefits.

### Summary of the Invention

In accordance with the present invention, controlled release of the drug with improved bioavailability, reduction in dose, reduction in dosage frequency, reduction in undesirable side effects and improved patient compliance are achieved by combining solubilization of low solubility drugs with gastro-retention.

Thus according to an aspect of the present invention there is provided a controlled release oral pharmaceutical composition comprised of a therapeutically effective amount of one or more pharmacologically active agent having low bioavailability; one or more solubilizers wherein the ratio of drug to said solubilizer is 20:1 to 1:20; one or more biocompatible swelling agents; and a swelling enhancer in an amount of 5 to 90 weight percent; wherein the swelling agent, in combination with swelling enhancer, swells in the presence of water in gastric fluid such that the size of the dosage form is sufficiently increased to provide retention of the dosage form in the stomach of a patient, and gradually erode within the gastrointestinal tract over a prolonged time period.

An object of the present invention is to provide controlled release pharmaceutical compositions for oral administration having at least one active pharmaceutical ingredient of low bioavailability due to low aqueous solubility and/or limited absorption in the gastrointestinal tract wherein its instantaneous solubility is increased prior to controlling its release.

Another object of the present invention is to solubilize low solubility drugs and further utilize the solubilized drugs to formulate controlled release compositions to effectively increase their bioavailability.

Yet another object of the present invention is to provide a simple and cost effective controlled release pharmaceutical composition, for improved bioavailability which would be simple and cost efficient to manufacture on a commercial scale.

A further object of the present invention is to provide gastroretentive compositions that are retained in the stomach for a longer period of time thereby increasing the bioavailability of drugs with limited absorption.

Another object of the present invention is to provide a controlled release pharmaceutical composition that has reduced level of dose frequency and therefore improved patient compliance.

Yet a further object of the present invention is to combine increased solubilization of drug with greater gastro-retention achieving controlled release of a low solubility drug, improved bioavailability, reduction in dose level, reduction in dosage frequency, reduction in undesirable side effects and improved patient compliance.

Another object of the invention is to provide a multi-layered tablet having either an instant release layer and a gastroretentive sustained release layer, or one or more gastroretentive sustained release layers.

Yet further object of the present invention is to provide a gastro-retentive composition which has increased solubility and the release of drug near absorption site to ensure better absorption of the drug resulting in increased bioavailability which coupled with extended release would result in the reduction in dose, dosage frequency, improved patient compliance and more importantly enhanced therapeutic benefits.

According to another aspect of the present invention there is provided a gastro retentive oral pharmaceutical dosage form in the form of an expanding multilayered system comprising an instant release layer having one or more active ingredients in a solubilized form and at least one additional layer having one or more active ingredients for controlled drug delivery, one or more solubilizers, one or more biocompatible swelling agents and a swelling enhancer.

According to another aspect of the present invention there is provided a gastro retentive oral pharmaceutical dosage form in the form of an expanding multilayered system comprising two or more sustained release layers having one or more active ingredients in a solubilized form in each layer. Each of the sustained release layers contains one or more active ingredients for controlled drug delivery, one or more solubilizers, one or more biocompatible swelling agents and a swelling enhancer.

Another object of the present invention is to provide a gastro-retentive composition with a solubilizer and a swelling enhancer, in the form of an expanding multilayered system for oral administration. The composition is adapted to deliver an active agent from a first layer immediately upon reaching the gastrointestinal tract and deliver same or different agent from a second layer, in a controlled manner over a specific time period, the second layer is also adapted to provide expanding dosage form, thereby effectively retaining the dosage form in the stomach.

### Brief Description of the Drawings

The foregoing and other features and advantages of the present invention will be more fully understood from the following detailed description of illustrative embodiments, taken in conjunction with the accompanying drawings in which:

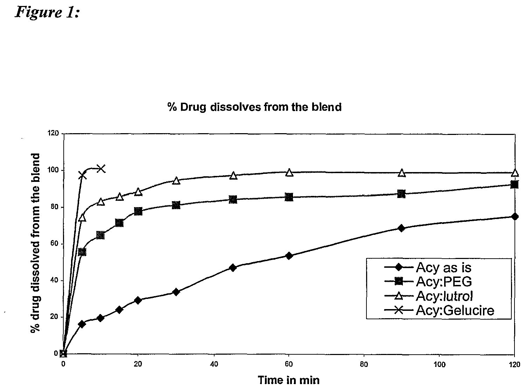

FIG. 1 is a graphic depiction of an in vitro dissolution study of acyclovir solubilized using various solubilizers;

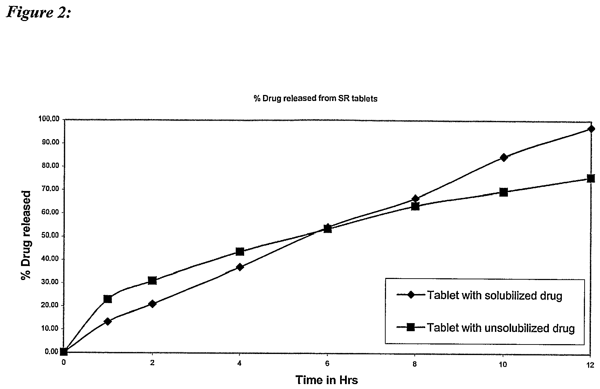

FIG. 2 is a graphic depiction of In vitro dissolution of Acyclovir tablets; and
FIG. 3 graphically shows that incorporation of a solubilizer increases dissolution rate of the acyclovir, which results in an increase in bioavailability.

### Detailed Description of Invention

The present invention comprises the preparation and use of a solubilized low solubility drug in a sustained release, gastro-retentive system wherein the maximum amount of drug will be available for absorption by virtue of its solubilized property and continuous release through the gastro-retentive system.

The invention is particularly useful for drugs having a narrow therapeutic window of absorption wherein gastro-retention employed according to the invention allows a continuous trickling of solubilized drug thereby maximizing bioavailability of the drug. Accordingly the present invention provides for two components for formulating the controlled release composition:
***Solubilization of the drug*:** - The low solubility drugs are solubilized using surface active agents like hydrophilic surfactants, lipophilic surfactants or mixtures thereof.
***Gastro-retention of the drug*:*-*** The solubilized drug is then incorporated in a gastro-retentive matrix system, which remains in the stomach by virtue of its size after swelling and allows a slow and continuous release of the solubilized drug which helps in increasing the extent of drug absorption and improving bioavailability.

### SOLUBILIZATION OF THE DRUG:

According to this invention, the increase in instantaneous solubility of the drug is achieved by using one or more suitable solubilizers. The low solubility drug and one or more solubilizers may be employed in different ratios. The selection of ratio depends upon the properties of the active ingredient, the desired improvement in its solubility and the type of solubilizers employed. It is contemplated within the scope of the invention that the ratio of drug: solubilizers can range from about 20 :1 to about 1:20. The preferred ratio of drug: solubilizers ranges from about 10 :1 to about 1 :10. The most preferred ratio being about 5:1 to about 1:5. A combination of solubilizers may also be included wherein the total amount of solubilizer employed is maintained in the above-mentioned ratios.

Different non-limiting processes may be employed to prepare a solid solution of the drug and solubilizer or to form a physical mixture so as to increase the solubility of the active ingredient. It is contemplated within the scope of the invention that the processes may include solubilization using melt granulation or solvent treatment method. In case of melt granulation, the solubilizer is melted and the drug is added and mixed with the molten mass effectively, allowed to solidify and the granules are separated from each other. In another illustrative embodiment of this system the drug is granulated using molten solubilizer. In some cases drug and solubilizer both may be melted together and cooled to room temperature.

In using a solvent treatment method, either the solubilizers or the drug, or both are dissolved in a solvent and the solvent is then evaporated. The resultant mass is a blend of drug and solubilizer, such that the solubility of the drug is increased. Solvent employed in this system may be aqueous or non-aqueous depending on the solubility of the drug and solubilizer.

It is contemplated within the scope of the invention that a combination of hot melt process and solvent treatment method can be employed. In this case the drug may be initially granulated with one or more molten solubilizer which can be further treated with a same /different solubilizer in a solvent or visa versa.

It is also contemplated within the scope of the invention that any process known in the art suitable for solubilization of drugs may be employed for the purpose of this invention.

Melt granulation and intimate physical mixture are the most preferred methods for solubilization of the drug, according to this invention. The increase in solubility can be determined by studying the actual solubility studies of the drug in presence of solubilizer or it can also be determined by carrying out dissolution studies in an appropriate dissolution medium. The dissolution method is preferred as it allows for calculation of the rate of dissolution by determining the amount of drug dissolved at different time intervals

### GASTRO-RETENTION OF DRUG:

According to the invention an additional component of the inventive system comprises increased gastro-retention. A number of gastro-retentive sustained release systems are reported in the literature. The following three major approaches describe gastroretentive controlled release devices that may be employed according to the invention:
***Floating or buoyant system:*** These systems have low density enabling them to float on gastric contents after their administration until the system either disintegrates, or the device absorbs fluid to the point where its density increases to an extent that it looses buoyancy and can then pass more easily from the stomach;
***Bioadhesive system**:-* This system is designed to imbibe fluid following their administration such that the outer layer becomes a viscous, tacky material that adheres to the gastric mucous/ mucus layer; and
***Swelling and expanding system:** -* These systems are designed to be sufficiently small on administration allowing for easy ingestion, but after ingestion rapidly swell or unfold to a size that precludes passage through the pylorus until after drug release has occurred.

Floating or buoyant systems require special techniques to decrease density of the dosage form or contain certain gas generating agent. These systems therefore are larger in size and do not allow use of high dosages of drugs. It is difficult to achieve bioadhesion in the gastric mucosa due to the large amount of fluid present in the stomach and also the gastric motility through the housekeeper wave that causes dislodgement of the dosage form. In one illustrative embodiment according to the invention a swelling and expanding system is employed. It is contemplated within the scope of the invention that other approaches for gastro-retention, namely floating and bioadhesive system or the like may be used.

In a first illustrative embodiment, a controlled release, gastro-retentive swelling system incorporating solubilized drug is contemplated. The controlled release gastro-retentive swelling system according to the invention employs a combination of polymers, which swell voluminously in the presence of gastric contents to increase the dosage form size such that it precludes its passage through the pylorus.

According to the invention it has been surprisingly found that addition of swelling enhancers to the gastro-retentive swelling system reduces the swelling time considerably which can further aid in improving bio-availability of drugs with narrow therapeutic absorption window.

The dosage form of the present invention is a solid dosage form, preferably a tablet, which may vary in shape including but not limited to oval, triangle, almond, peanut, parallelogram, pentagonal. It is contemplated within the scope of the invention that the dosage form can be encapsulated.

Tablets in accordance with the invention may be manufactured using conventional techniques of common tableting methods known in the art such as direct compression, wet granulation, dry granulation and extrusion/ melt granulation.

The dosage form according to the invention may include excipients conventionally known in art such as filler, binders and lubricants. Fillers such as lactose monohydrate, microcrystalline cellulose, dicalcium phosphate or the like may be used. Binders like polyvinyl pyrolidone (PVP), copovidone or the like may be used. Lubricants such as Aerosil-200, magnesium stearate and hydrogenated vegetable oils and triglycerides of stearic acid, palmitic acid or the like may be utilized.

In one illustrative embodiment according to the invention, the dosage form may be optionally coated. Surface coatings may be employed for aesthetic purposes or for dimensionally stabilizing the compressed dosage form. The surface coating may be any conventional coating which is suitable for enteral use. The coating may be carried out using any conventional technique employing conventional ingredients. A surface coating can for example be obtained using a quick-dissolving film using conventional polymers such as hydroxypropyl methyl cellulose, hydroxypropyl cellulose, carboxymethyl cellulose, polyvinyl alcohol poly methacrylates or the like.

In a further illustrative embodiment a solid pharmaceutical composition in the form of an expanding multilayer system for oral administration is adapted to deliver an active pharmaceutical agent from a first layer immediately upon reaching the gastrointestinal tract, and to deliver a further pharmaceutical agent which may be same or different from a second layer, in a controlled manner over a specific time period. The second layer is also adapted to provide expanding nature for the dosage system, thereby making the dosage system have greater retention in the stomach.

In this further illustrative embodiment a solid pharmaceutical composition for oral administration contains two or more layers comprising of an instant release (IR) layer comprising an active ingredient, filler such as lactose, microcrystalline cellulose and disintegrant such as croscarmellose sodium, a lubricant such as magnesium stearate, and optionally other excipients and other active ingredients. The pharmaceutical active agent in this instant release layer may be present in a solubilized form.

The pharmaceutical composition according to this illustrative embodiment further contains at least one second layer, which is referred to as a controlled release layer (CRL) that includes one or more pharmaceutical active agent for controlled drug delivery, one or more solubilizers, one or more biocompatible swelling agent and a swelling enhancer. The swelling agent, in combination with swelling enhancer, swells in presence of water in gastric fluid such that the size of the dosage form is sufficiently increased to provide retention of the dosage form in the stomach of a patient, and gradually erode within the gastrointestinal tract over a prolonged time period.

The disintegrating agent present in the first layer (IR) can be selected from a group including but not limited to the following: starch, sodium starch glycolate, pregelatinised starch, crosslinked poly vinyl pyrrolidone, cross linked carboxy methyl cellulose, ion exchange resin, the most preferred being sodium starch glycolate. Sodium starch glycolate is present in an amount ranging from about 0.25% to about 10%, more preferably about 0.5 to about 5.0% and most preferably about 1% by weight based on the total weight of the composition.

Each of these layers may contain an active pharmaceutical ingredient, with the ratio of the active ingredient in the first layer (IR) to the active ingredient in the second layer (CRL) being in the range of from about 10:90 to about 90:10 by weight. It is contemplated within the scope of the invention that these layers may contain the same or different active pharmaceutical ingredients such that one of the active ingredient is in the form of instant release dosage form whereas the other may be in the controlled release form.

In a further illustrative embodiment a solid pharmaceutical composition in the form of an expanding multilayer system for oral administration is adapted to deliver at least two active agents present in different layers in a controlled manner over a specific time period.

The dosage form having either a single layer or multi-layer composition will after ingestion gradually swell upon contact with gastric fluid. The time taken for swelling may vary from about 15 min to about 4 hours preferably within about 15 min to about 3 hours and most preferably within about 15 min to about 2 hours. The shorter axis of the dosage form has to expand to a length of more than about 0.8 cm and preferably more than about 1.0 cm.

### Pharmacologically active agent:

The pharmacologically active agents according to the invention are those having low bioavailability. It is contemplated within the scope of the invention, however, that any pharmaceutical active ingredient may be used. The low bioavailability can be because of low solubility and/or limited oral absorption or a narrow therapeutic absorption window. The active agents may be selected, but not limited to, one of the following therapeutic classes of active substances that includes: antiulcer, antidiabetic, anticoagulant, antithrombic, hypolipaemic, antiarrhythmic, vasodilatory, antianginal, antihypertensive, and vasoprotective agents, fertility enhancers, labour inducers and inhibitors, and contraceptive, antibiotic, antifungal, antiviral, anticancer, anti-inflammatory, analgesic, antiepileptic, antiparkinsonian, neuroleptic, hypnotic, anxiolytic, psychostimulatory, antimigraine, antidepressant, antitussive, antihistamine and antiallergic agents.

The active pharmaceutical agents may be selected, but not limited to, pentoxifylline, prazosin, acyclovir, levodopa, nifedipine, diltiazem, naproxen, flurbiprofen, ketoprofen, fenoprofen, fentiazac, oestradiol valerate, metoprolol, sulpiride, captopril, cimetidine, zidovudine, nicardipine, terfenadine, salbutamol, carbamazepine, ranitidine, enalapril, simvastatin, fluoxetine, famotidine, ganciclovir, famiciclovir, valaciclovir ciprofloxacin pentazocine, omeprazole, saquinavir, ritonavir, nelfinavir, thiamphenicol, clarithromycin, azithromycin, ceftazidime, cyclosporine, digoxin, paclitaxel, iron salts, eprosartan, losartan potassium, valsartan, candesartan, topiramate, ketoconazole and mixtures thereof.

### Solubilizer:

In accordance with features of the present invention, the solubilizer acts to increase the instantaneous solubility of the pharmaceutically active agent. The solubilizer may be selected from hydrophilic surfactants or lipophilic surfactants or mixtures thereof. The surfactants may be anionic, nonionic, cationic, and zwitterionic surfactants.

The hydrophilic non-ionic surfactants may be selected from the group comprised of, but not limited to: polyethylene glycol sorbitan fatty acid esters and hydrophilic transesterification products of a polyol with at least one member of the group consisting of triglycerides, vegetable oils, and hydrogenated vegetable oils preferably glycerol, ethylene glycol, polyethylene glycol, sorbitol, propylene glycol, pentaerythritol, or a saccharide, d-α-tocopheryl polyethylene glycol 1000 succinate.

The ionic surfactants may be selected from the group comprised of, but not limited to: alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acyl lactylates; mono- and di-acetylated tartaric acid esters of mono- and di-glycerides; succinylated mono- and di-glycerides; citric acid esters of mono- and di-glycerides; and mixtures thereof.

The lipophilic surfactants may be selected from the group comprised of, but not limited to: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; PEG sorbitan fatty acid esters, PEG glycerol fatty acid esters, polyglycerized fatty acid, polyoxyethylene-polyoxypropylene block copolymers, sorbitan fatty acid esters; and mixtures thereof.

Preferably the solubilizer may be selected from PEG-20-glyceryl stearate (Capmul® by Abitec), PEG-40 hydrogenated castor oil (Cremophor RH 40® by BASF), PEG 6 corn oil (Labrafil® by Gattefosse), lauryl macrogol - 32 glyceride (Gelucire 44/14® by Gattefosse) stearoyl macrogol glyceride (Gelucire 50/13® by Gattefosse), polyglyceryl - 10 mono dioleate (Caprol ® PEG 860 by Abitec), propylene glycol oleate (Lutrol ® by BASF), Propylene glycol dioctanoate (Captex® by Abitec)Propylene glycol caprylate/caprate (Labrafac® by Gattefosse), Glyceryl monooleate (Peceol® by Gattefosse), Glycerol monolinoleate (Maisine ® by Gattefosse), Glycerol monostearate (Capmul® by Abitec), PEG- 20 sorbitan monolaurate (Tween 20® by ICI), PEG - 4 lauryl ether (Brij 30® by ICI), Sucrose distearate (Sucroester 7® by Gattefosse), Sucrose monopalmitate (Sucroester 15® by Gattefosse), polyoxyethylene-polyoxypropylene block copolymer (Lutrol® series BASF), polyethylene glycol 660 hydroxystearate, (Solutol® by BASF), Sodium lauryl sulphate, Sodium dodecyl sulphate, Dioctyl suphosuccinate, L- hydroxypropyl cellulose, hydroxylethylcellulose, hydroxy propylcellulose, Propylene glycol alginate, sodium taurocholate, sodium glycocholate, sodium deoxycholate, betains , polyethylene glycol (Carbowax® by DOW), d-α-tocopheryl polyethylene glycol 1000 succinate. (Vitamin E TPGS® by Eastman) and mixtures thereof.

A more preferred solubilizer may be selected from PEG-40 hydrogenated castor oil (Cremophor RH 40® by BASF), lauryl macrogol - 32 glyceride (Gelucire 44/14® by Gattefosse) stearoyl macrogol glyceride (Gelucire 50/13® by Gattefosse), PEG- 20 sorbitan monolaurate (Tween 20® by ICI), PEG - 4 lauryl ether (Brij 30® by ICI), polyoxyethylene-polyoxypropylene block copolymer (Lutrol® series BASF), Sodium lauryl sulphate, Sodium dodecyl sulphate, polyethylene glycol (Carbowax® by DOW) and mixtures thereof.

### Biocompatible swelling agent:

The swelling agent used in the present invention includes one or more swellable biocompatible hydrophilic polymers. Preferably, the polymers are employed in the dry state or in a form that has substantial capacity for water uptake.

Water-soluble polymers used as swelling agents that are useful in preparation of the said composition of this invention are polymers that are nontoxic and swell in a dimensionally unrestricted manner upon imbibition of gastric fluid. Examples of polymers which can be used include but are not limited to: polyalkylene oxides; cellulosic polymers; acrylic acid and methacrylic acid polymers, and esters thereof, maleic anhydride polymers; polymaleic acid; poly(acrylamides); poly(olefinic alcohol)s; poly(N-vinyl lactams); polyols; polyoxyethylated saccharides; polyoxazolines; polyvinylamines; polyvinylacetates; polyimines; starch and starch-based polymers; polyurethane hydrogels; chitosan; polysaccharide gums; zein; shellac-based polymers; polyethylene oxide, hydroxypropyl cellulose, hydroxypropyl methyl cellulose, hydroxyethyl cellulose, sodium carboxy methylcellulose, calcium carboxymethyl cellulose, methyl cellulose, polyacrylic acid, maltodextrin, pre-gelatinized starch and polyvinyl alcohol, copolymers and mixtures thereof.

One or more hydrophilic polymers are preferably selected from the group consisting of polyethylene oxide, hydroxypropyl cellulose, hydroxypropyl methyl cellulose, hydroxyethyl cellulose, sodium carboxy methylcellulose, calcium carboxymethyl cellulose, methyl cellulose, polyacrylic acid, maltodextrin, pre-gelatinized starch, polyvinyl alcohol and mixtures thereof.

One or more hydrophilic polymers are more preferably a polyalkylene oxide selected from the group consisting of poly(ethylene oxide), poly(ethylene oxide-co-propylene oxide), and mixtures thereof.

One or more hydrophilic polymers are most preferably poly(ethylene oxide). At least one of the biocompatible hydrophilic polymer has an average molecular weight in the range of about 5,000 to about 20,000,000.

The weight percent of the hydrophilic polymer in the dosage form is about 5 to about 90 weight percent, preferably about 10 to about 70 weight percent, and most preferably about 15 to about 50 weight percent.

### Swelling enhancers:

Swelling enhancers are members of a special category of excipients that swell rapidly to a large extent resulting in a dramatic increase in the size of the tablet. At lower concentrations, these excipients are used as superdisintegrants; however at concentration above 5 % w/w these agents function as swelling enhancers and help increase the size of the dosage form.

According to the invention swelling enhancers include but are not limited to: low-substituted hydroxypropyl cellulose, microcrystalline cellulose, cross-linked sodium or calcium carboxymethyl cellulose, cellulose fiber, cross-linked polyvinyl pyrrolidone, cross-linked polyacrylic acid, cross-linked Amberlite resin, alginates, colloidal magnesium-aluminum silicate, corn starch granules, rice starch granules, potato starch granules, pregelatinised starch and sodium carboxymethyl starch.

According to the invention the swelling enhancer is preferably cross-linked polyvinyl pyrrolidone. The content of the swelling enhancer is about 5 to about 90 weight percent preferably about 10 to about 70 weight percent, most preferably about 15 to about 50 weight percent.

The composition according to the invention may make use of a single polymer alone or a combination of polymers with or without a swelling enhancer as required. When a combination of polymers and a swelling enhancer is employed for gastro-retention, the swelling enhancer allows a rapid and dramatic increase in the size of the tablets.

The swelling enhancer cannot maintain the integrity of the dosage form and avoid its disintegration and polymers may not show the rapid increase in size desired for gastro-retention by themselves alone due to their slow rate of swelling. Therefore, a synergistic combination may preferably be employed which allows rapid swelling by virtue of the presence of swelling enhancer and maintenance of integrity by polymeric network formed by swelling of the polymer(s).

Thus the invention describes a unique combination of technologies wherein a solubilized drug is incorporated into a swelling matrix of polymer(s) and swelling enhancer to achieve gastro-retention. Controlled release is thus achieved by; integrity of the matrix and the need for the gastric fluid to diffuse into the matrix or is achieved by controlled rate of erosion of the matrix, and the need for the matrix to erode in order to release much of the drug or a combination of the two.

The gastro-retentive controlled release compositions according to the invention includes a solubilized drug that finds utility when administered to patients in the fed or the fasting mode. The fed mode is preferred since the narrowing of the pyloric opening that occurs in the fed mode serves as a further means of promoting gastric retention by retaining a broader range of size of the dosage form. Following oral administration to a patient, the dosage form is retained in the upper gastrointestinal tract for a time period of about 30 min to about 12 hours or about 1 hour to about 9 hours or most preferably about 1 hour to about 6 hours.

While the present invention has been described in terms of its specific illustrative embodiments, certain modifications and equivalents will be apparent to those skilled in the art and are intended to be included within the scope of the present invention. The details of the invention, its objects and advantages are explained hereunder in greater detail in relation to non-limiting exemplary illustrations

### Example 1:

### Swelling studies:

In this example various polymer placebo tablets were prepared at a polymer concentration of 20% w/w and the rate of swelling was determined in 0.1N HCl

**Table 1**

| ***Swelling of various polymer tablets*** | | | | | | |
|---|---|---|---|---|---|---|
| **Sr. No** | **Polymers** | **Swelling in 50 ml 0.1 N HCl** | | | | |
| | | 15 min | 1 Hrs | 2 Hrs | 3 Hrs | 4 hrs |
| 1. | Xanthan Gum | 18.8x 8 mm | 20x10mm | 21x11mm | 21x12mm | 21x12mm |
| 2. | Polyethylene oxide (Sentry Polyox WSR 1105) | 18.8x 8 mm | 20x10mm | 21x11 mm with slight erosion | 21x12mm with slight erosion | 21x12 mm with erosion |
| 3. | Polyethylene oxide (Sentry Polyox WSR 60K) | 18.8x 8 mm | 20x10mm | 21x12 mm | 21x13 mm | 21x13 mm |
| 4. | Polyethylene oxide (Sentry Polyox WSR 301) | 18.8x 8 mm | 20x10mm | 21x12 mm | 21x12 mm | 21x13 mm |
| 5. | Hydroxypropyl methylcellulose (Methocel K100) | 18.8x 8 mm | 19x9 mm | 20x10 mm | 21x11 mm | 22x12 mm |
| 6. | Hydroxypropyl methylcellulose (Methocel K100 M) | 18.8x 8 mm | 19x9 mm | 20x10 mm | 21x11 mm | 22x12 mm |
| 7. | Hydroxypropyl methylcellulose (Methocel K4M) | 18.8x 8 mm | 19x10 mm | 20x10 mm | 21x11 mm | 22x12 mm |

The study showed that among various polymers polyoxyethylenes exhibited a maximum rate of swelling. Although, these polymers alone can be used for gastro-retentive drug delivery systems, there is a need to further increase the rate of swelling.

### Example 2:

### Swelling studies of tablets containing Swelling Enhancers

In this example swelling enhancers, namely crospovidone, crosscarmellose sodium, sodium starch glycolate and starch 1500, were incorporated into a placebo tablet at a concentration of about 10% w/w. However these agents resulted in too rapid and voluminous swelling of the dosage forms leading to their disintegration.

### Example 3:

### Swelling studies of tablets containing combination ofpolymers and swelling enhancers

A combination of swelling enhancer and a matrix forming polymer were incorporated in a placebo tablet. Table 2 shows the rates of swelling for these dosage forms.

**Table: 2**

| ***Swelling data of tablets containing combination ofpolymers and swelling enhancers*** | | | | |
|---|---|---|---|---|
| **Sr. No.** | **Polymer / swelling enhancer** | **15 min** | **60 min** | **120 min** |
| 1 | Polyethylene oxide (Sentry Polyox WSR 60K) / Crospovidone (1:1.5) | 18.8x 8 mm | 22 x12 mm with erosion | 22 x13 mm with erosion |
| 2 | Polyethylene oxide (Sentry Polyox WSR 60K) /Crospovidone (1:1) | 18.8x 8 mm | 22 x13 mm with slight erosion | 22 x13 mm with slight erosion |
| 3 | Polyethylene oxide (Sentry Polyox WSR 60K) / Crospovidone (1.5:1) | 18.8x 8 mm | 22 x13 mm with slight erosion | 22 x13 mm with slight erosion |
| 4 | Hydroxypropyl methylcellulose (Methocel K100M)/Crospovidone (1.5 :1) | 18.8x 8 mm | 20 x 10 mm | 21 x 11 mm |
| 5 | Hydroxypropyl methylcellulose (Methocel K4M) / Crospovidone (1.5 :1) | 18.8x 8 mm | 20 x 11 mm | 22 x 11 mm |

Example 3 shows that the combination of a swelling enhancer and polymer results in dosage form with a faster rate of swelling, as desired for gastro-retention.

### Example 4:

### Solubilization of drug using various solubilizing agents:

A solublizing agent was melted in a container and a drug was added and mixed intimately and cooled to room temperature. The mass was sifted through an appropriate sieve to get a uniform blend. A blend of the drug was prepared using polyethylene glycol 6000, Lutrol F127 and Gelucire (50/13). Solid dispersion of the drug with various solubilizing agents like polyethylene glycol 6000, Lutrol F127 and Gelucire 50/13 were studied for their solubility in 900 ml distilled water.

Acyclovir in a ratio of (1:1 and 1:5) with polyethylene glycol 6000 showed a two-fold increase in solubility, acyclovir in ratio of (1:0.5 to 1:1) with Gelucire 50/13 showed a 5 fold increase in instantaneous solubility against acyclovir as such. Also with Lutrol in ratio (1:0.5 to 1:2) a three-fold increase in instantaneous solubility was observed. These samples were taken for dissolution study and the result obtained are provided in Table 3 hereunder and graphically depicted in Figure-I:

**Table 3:**

| ***In vitro dissolution study of acyclovir solubilized using various solubilizers*** | | | | |
|---|---|---|---|---|
| Time in min | Acyclovir as is (% drug dissolved) | Acyclovir :PEG (% drug dissolved) | Acyclovir : Lutrol (% drug dissolved) | Acyclovir: Gelucire %dissolved |
| 0 | 0 | 0 | 0 | 0 |
| 5 | 16.03 | 55.38 | 74.34 | 97.25 |
| 10 | 19.58 | 64.79 | 83.16 | 100.96 |
| 15 | 24.09 | 71.46 | 85.77 | |
| 20 | 29.34 | 77.85 | 88.49 | |
| 30 | 33.89 | 81.32 | 94.57 | |
| 45 | 47.11 | 84.30 | 97.22 | |
| 60 | 53.78 | 85.64 | 99.06 | |
| 90 | 68.87 | 87.45 | 98.86 | |
| 120 | 75.40 | 92.67 | 99.13 | |

As would be evident from the above data, we may conclude that use of solubilizing agents increases the instantaneous solubility of the low-solubility drugs like Acyclovir.

### Example 5:

### Gastroretentive tablets of Acyclovir

The solubilized drug was further used for formulating controlled release tablets. Based on the solubility data it was decided to use the combination of Acyclovir : Gelucire 50 /13 for the preparation of the tablets.

**Table 4:**

| ***Composition of acyclovir tablets with and without solubiliser*** | | |
|---|---|---|
| Ingredients | A (Mg/tablet) | B (Mg/tablet) |
| Acyclovir | 250.00 | 250.00 |
| Stearoyl macrogol glyceride (Gelucire 50/13®) | 50.00 | - |
| Polyethylene oxide (Sentry Polyox WSR 60K) | 300.00 | 300.00 |
| Crospovidone | 350.00 | 350.00 |
| Polyvinyl pyrrolidone K30 (PVP K30) | 50.00 | 50.00 |
| Magnesium stearate | 10.00 | 10.00 |

Gelucire was melted and acyclovir was granulated with molten gelucire. These granules of acyclovir were further granulated with polymers using PVP K30. Granules were dried and lubricated and further compressed into tablets using a compression machine. In case of formulation B drug was mixed with polymers and granulated and similar procedure was further followed.

**Dissolution condition:**

| | |
|---|---|
| Dissolution medium | : 0.1N HCl |
| Volume of the dissolution medium | : 900 ml |
| Temperature | : 37°C |

The results obtained are represented hereunder in Table V and graphically depicted in Figure-II:

**Table 5:**

| ***In vitro dissolution of Acyclovir tablets*** | | |
|---|---|---|
| Time intervals (hr) | Tablet with solubilized drug | Tablet with unsolubilized drug |
| 0 | 0.00 | 0.00 |
| 2 | 20.89 | 30.95 |
| 4 | 36.90 | 43.57 |
| 8 | 66.73 | 63.39 |
| 10 | 84.59 | 69.72 |
| 12 | 97.04 | 75.81 |
| 14 | - | 78.04 |

### Example 6

### In vivo study

In vivo study was carried out to determine the relative bioavailability of Acyclovir from the test formulation of Example 5 (Acyclovir 250 mg tablets) in comparison to the reference formulation Zovirax® (Acyclovir 200 mg tablets). The study was open label, balanced, randomized, two-treatment, two-period, two-sequence, single dose, crossover, and comparative oral bioavailability study in healthy, adult, male human subjects (n=4) under non-fasting conditions. The blood levels were monitored over 24 hours time period.

**Table 6**

| **In vivo data** | | |
|---|---|---|
| No. | Formulation | AUC_{0→∝} (ng.h/mL) |
| 1 | Reference | 2624.96 |
| 2 | Test product | 5920.23 |

The data indicate that there is significant increase in the bioavailability of the formulation of the present invention compared to the reference product.

### Example 7:

### Azithromycin formulation

**Table 7**

| ***Composition of Azithromycin tablets*** | |
|---|---|
| **Ingredients** | **Mg/tablet** |
| Azithromycin | 250.00 |
| Polyoxyethylene polypropylene block copolymer (Lutrol F68) | 125.00 |
| Hydroxy propyl methylcellulose (Methoce1 K100M) | 80.00 |
| Hydroxyethyl cellulose (Natrosol HF) | 80.00 |
| Sodium starch glycolate (Primojel) | 200.00 |
| Microcrystalline cellulose (Avicel PH102) | 250.00 |
| Polyvinyl pyrrolidone K30 (PVP K30) | 50.00 |
| Magnesium stearate | 10.00 |

Lutrol was melted and azithromycin was added to the molten Lutrol forming a dispersion. The dispersion was mixed and cooled while mixing to achieve a homogenous mass. Granules of azithromycin were further granulated with polymers using PVP K30. Granules were dried and lubricated and further compressed into tablets using a compression machine.

### Example 8

### Simvastatin formulation

**Table 8**

| ***Composition of Simvastatin tablets*** | |
|---|---|
| **Ingredients** | **Mg/tablet** |
| Simvastatin | 80.00 |
| Polyethylene glycol 6000 (Carbowax 6000) | 160.00 |
| Sodium carboxymethyl cellulose (Cekol 30000) | 150.00 |
| L-Hydroxy propyl cellulose (L-HPC) | 130.00 |
| Dicalcium phosphate | 200.00 |
| Lactose | 250.00 |
| Polyvinyl pyrrolidone | 50.00 |
| Magnesium stearate | 10.00 |

Polyethylene glycol was melted and simvastatin was added to the molten Polyethylene glycol forming a dispersion. The dispersion was mixed and cooled while mixing to achieve a homogenous mass. Granules of drug were further granulated with polymers using PVP K30. Granules were dried and lubricated and further compressed into tablets using a compression machine.

### Example 9

### Carbamazepine formulation

**Table 9**

| ***Composition of Carbamazepine tablets*** | |
|---|---|
| **Ingredients** | **Mg/tablet** |
| Carbamazepine | 200.00 |
| Sodium lauryl sulphate | 50.00 |
| PEG 40 hydrogenated castor oil (Cremophor RH40) | 50.00 |
| Hydroxy propyl methylcellulose (Methocel K100M) | 90.00 |
| Sodium carboxymethyl cellulose (Cekol 30,000) | 45.00 |
| Alginic acid | 135.00 |
| Silicified microcrystalline cellulose (Prosolve 90) | 400.00 |
| Polyvinyl pyrrolidone K30 (PVP K30) | 50.00 |
| Magnesium stearate | 10.00 |

Cremophor RH 40 was melted and sodium lauryl sulphate was dispersed in it and carbamazepine was added forming a dispersion. The dispersion was mixed and cooled while mixing to achieve a homogenous mass. Granules of drug were further granulated with polymers using PVP K30. Granules were dried and lubricated and further compressed into tablets using a compression machine.

### Example 10: Bilayered tablets of Acyclovir with and without solubilizer

### Acyclovir SR component

**Table 10: Pomposition of Acyclovir tablet sustained release layer**

| **Ingredients** | **Mg/tablet** | |
|---|---|---|
| | A | B |
| Acyclovir | 200.00 | 200.00 |
| Stearoyl macrogol glyceride (Gelucire 50/13®) | 40.00 | |
| Polyethylene oxide (Sentry Polyox WSR 60K) | 240.00 | 240.00 |
| Crospovidone | 280.00 | 280.00 |
| Polyvinyl pyrolidone 30 K | 50.00 | 50.00 |
| Dextrates Dihydrate | 220.00 | 220.00 |
| Magnesium Stearate | 10.00 | 10.00 |

### Acyclovir IR component

**Table 11:Composition of Acyclovir tablet immediate release layer**

| **Ingredients** | **Mg/tablet** |
|---|---|
| Acyclovir | 50.00 |
| Microcrystalline cellulose (Avicel PH101) | 52.50 |
| Polyvinyl pyrolidone 30 K | 2.00 |
| Sodium Starch Glycol ate (Primojel) | 5.00 |
| Magnesium Stearate | 0.5 |

### Preparation of sustained release granules (Formulation A):

Acyclovir was mixed with molten Gelucire50/13. This mixture was then blended with Polyethylene oxide WSR60K, Crospovidone, Dextrates Dihydrate. This blend was further granulated with Polyvinyl pyrolidone 30K. The granules were dried and lubricated with Magnesium Stearate.

### Reparation of sustained release granules (Formulation B):

Acyclovir was blended with Polyethylene oxide WSR60K, Crospovidone, Dextrates Dihydrate. This blend was further granulated with Polyvinyl pyrolidone 30K. The granules are dried and lubricated with Magnesium Stearate.

### Preparation of immediate release granules:

The IR component was prepared by granulating the drug along with microcrystalline cellulose using polyvinyl pyrolidone-30K and lubricating with Sodium Starch Glycolate and Magnesium Stearate.

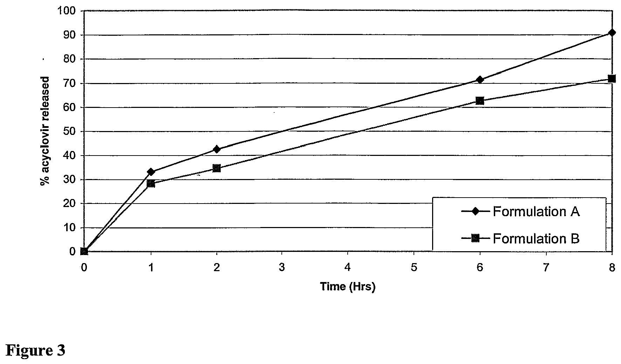

The SR component of formulation A and B and IR component were compressed together to form a double layer tablet. It is evident from Figure 3 that incorporation of a solubilizer increases dissolution rate of the acyclovir, which results in an increase in bioavailability.

It will be understood that various modifications may be made to the embodiments and examples disclosed herein. Therefore, the above description and examples should not be construed as limiting, but merely as exemplification of the various embodiments. Those skilled in the art will envision other modifications within the scope and spirit of the claims appended hereto.

## Claims

1. A controlled release oral pharmaceutical composition comprising of:
a. therapeutically effective amount of one or more pharmacologically active agents showing low bioavailability;
b. one or more solubilizers wherein ratio of drug to said solubilizer is 20:1 to 1 :20;
c. one or more biocompatible swelling agents; and
d. a swelling enhancer in an amount of 5 to 90 weight percent ;
wherein the swelling agent in combination with swelling enhancer, swell in the presence of gastric fluid such that the size of the dosage form is sufficiently increased to provide retention of the dosage form in the stomach of a patient, and gradually erode within the gastrointestinal tract over a prolonged time period.

2. The controlled release oral pharmaceutical composition of claim 1, wherein the pharmacologically active agent is selected from the group consisting of: antiulcer, antidiabetic, anticoagulant, antithrombic, hypolipaemic, antiarrhythmic, vasodilatory, antianginal, antihypertensive, vasoprotective agents, fertility enhancers, labour inducers and inhibitors, contraceptive, antibiotic, antifungal, antiviral, anticancer, anti-inflammatory, analgesic, antiepileptic, antiparkinsonian, neuroleptic, hypnotic, anxiolytic, psychostimulatory, antimigraine, antidepressant, antitussive, antihistamine or antiallergic agents.

3. The controlled release oral pharmaceutical composition of claim 1 wherein the pharmacologically active agent is selected from the group consisting of pentoxifylline, prazosin, acyclovir, levodopa, nifedipine, diltiazem, naproxen, flurbiprofen, ketoprofen, fenoprofen, fentiazac, oestradiol valerate, metoprolol, sulpiride, captopril, cimetidine, zidovudine, nicardipine, terfenadine, salbutamol, carbamazepine, ranitidine, enalapril, simvastatin, fluoxetine, famotidine, ganciclovir, famiciclovir, ciprofloxacin, pentazocine, omeprazole, saquinavir, ritonavir, indinavir, nelfinavir, thiamphenicol, calcium carbonate, clarithromycin, azithromycin, ceftazidime, cyclosporine, digoxin, paclitaxel, iron salts, topiramate, and ketoconazole and mixtures thereof.

4. The controlled release oral pharmaceutical composition as of claim 1 wherein the pharmacologically active agent is acyclovir.

5. The controlled release oral pharmaceutical composition of claim 1, wherein the solubilizer is selected from the group consisting of hydrophilic surfactants, lipophilic surfactants and mixtures thereof.

6. The controlled release oral pharmaceutical composition as claimed in claim 1, wherein the solubilizer is selected from anionic, nonionic, cationic, and zwitterionic surfactants.

7. The controlled release oral pharmaceutical composition of claim 1, wherein the solubilizer comprises one or more hydrophilic nonionic surfactants selected from the group consisting of polyethylene glycol sorbitan fatty acid esters and hydrophilic transesterification products of a polyol with at least one member of the group consisting of triglycerides, vegetable oils, and hydrogenated vegetable oils.

8. The controlled release oral pharmaceutical composition of claim 1, wherein the solubilizer is selected from PEG-20-glyceryl stearate, PEG-40 hydrogenated castor oil, PEG 6 corn oil, lauryl macrogol - 32 glyceride, stearoyl macrogol glyceride, polyglyceryl 10 mono dioleate, propylene glycol oleate, Propylene glycol dioctanoate, Propylene glycol caprylate/caprate, Glyceryl monooleate, Glycerol monolinoleate, Glycerol monostearate, PEG- 20 sorbitan monolaurate, PEG - 4 lauryl ether, Sucrose distearate, Sucrose monopalmitate, polyoxyethylene-polyoxypropylene block copolymer, polyethylene glycol 660 hydroxystearate, Sodium lauryl sulphate, Sodium dodecyl sulphate, Propylene glycol alginate, sodium taurocholate, sodium glycocholate, sodium deoxycholate, betaines, polyethylene glycol and mixtures thereof

9. The controlled release oral pharmaceutical composition of claim 1, wherein the solubilizer is preferably a well-defined mixture of mono-, di- and triglycerides and mono- and di-fatty acid esters of polyethylene glycol.

10. The controlled release oral pharmaceutical composition of claim 1, wherein the ratio of drug to solubilizer is 10:1 to 1:10.

11. The controlled release oral pharmaceutical composition of claim 1, wherein the ratio of drug to solubilizer is 5:1 to 1:5.

12. The controlled release oral pharmaceutical of claim 1, wherein the biocompatible swelling agent is selected from the group consisting of: polyalkylene oxides; cellulosic polymers; acrylic acid and methacrylic acid polymers, and esters thereof, maleic anhydride polymers; polymaleic acid, poly(acrylamides); poly(olefinic alcohol)s; poly(N-vinyl lactams); polyols; polyoxyethylated saccharides; polyoxazolines; polyvinylamines; polyvinylacetates; polyimines; starch and starch based polymers; polyurethane hydrogels; chitosan; polysaccharide gums; zein; shellac- based polymers; and copolymers and mixtures thereof.

13. The controlled release oral pharmaceutical composition of claim 1, wherein the biocompatible swelling agent is one or more hydrophilic polymer selected from the group consisting of polyethylene oxide, hydroxypropyl cellulose, hydroxypropyl methyl cellulose, hydroxyethyl cellulose, sodium carboxy methylcellulose, calcium carboxymethyl cellulose, methyl cellulose, polyacrylic acid, maltodextrin, pre-gelatinized starch, polyvinyl alcohol and mixtures thereof.

14. The controlled release oral pharmaceutical composition of claim 1, wherein the poly alkylene oxide is one or more hydrophilic polymer selected from the group consisting of poly (ethylene oxide), poly(ethylene oxide-co- propylene oxide), and mixtures thereof.

15. The controlled release oral pharmaceutical composition of claim 14, wherein the hydrophilic polymer is poly(ethylene oxide).

16. The controlled release oral pharmaceutical composition of claim 1, wherein the biocompatible swelling agent is one or more hydrophilic polymer and the content of the hydrophilic polymer in the polymer matrix is 5 to 90 weight percent.

17. The controlled release oral pharmaceutical composition of claim 16, wherein the content of the hydrophilic polymer in the polymer matrix is 10 to 70 weight percent.

18. The controlled release oral pharmaceutical composition of claim 17, wherein the content of the hydrophilic polymer in the polymer matrix is 15 to 50 weight percent.

19. The controlled release oral pharmaceutical composition of claim 1, wherein the swelling enhancer is selected from the group consisting of low-substituted hydroxypropyl cellulose, microcrystalline cellulose, cross-linked sodium or calcium carboxymethyl cellulose, cellulose fiber, cross-linked polyvinyl pyrrolidone, cross-linked polyacrylic acid, cross- linked Amberlite resin, alginates, colloidal magnesium-aluminium silicate, corn starch granules, rice starch granules, potato starch granules, pregelatinised starch, sodium carboxymethyl starch and mixtures thereof.

20. The controlled release oral pharmaceutical composition of claim 1, wherein the swelling enhancer is selected from the group consisting of cross-linked sodium, calcium carboxymethyl cellulose, cross- linked polyvinyl pyrrolidone, sodium carboxymethyl starch, pregelatinised starch and mixtures thereof.

21. The controlled release oral pharmaceutical composition of claim 1, wherein the swelling enhancer is a cross-linked polyvinyl pyrrolidone.

22. The controlled release oral pharmaceutical composition of claim 1, wherein the content of the swelling enhancer is 10 to 70 weight percent.

23. The controlled release oral pharmaceutical composition of claim 1, wherein the content of the swelling enhancer is 15 to 50 weight percent.

24. The controlled release oral pharmaceutical composition of claim 1, wherein the composition is in the form of an expanding multi-layered system comprising a first layer having at least one active pharmaceutical ingredient with an immediate release property; and a second layer with a sustained release property having at least one active pharmaceutical ingredient showing low bioavailability, one or more solubilizers, one or more biocompatible swelling agents and a swelling enhancer.

25. The controlled release oral pharmaceutical composition according to claim 24 wherein the ratio of said active ingredient in said first layer to said active ingredient in said second layer is in the range of from 10:90 to 90:10 by weight.

26. The controlled release oral pharmaceutical composition according to claim 25 wherein said first layer further comprises a disintegrating agent selected from group consisting of starch, sodium starch glycolate, pregelatinised starch, crosslinked poly vinyl pyrrolidone, cross linked carboxy methyl cellulose, ion exchange resin and mixtures thereof.

27. The controlled release oral pharmaceutical composition according to claim 26 wherein said disintegrating agent is present in an amount ranging from 0.25% to 10%, more preferably 0.5 to 5.0% and most preferably 1% by weight based on the total weight of the composition.

28. A process for preparing a pharmaceutical composition comprising the steps of solubilizing an active pharmaceutical active ingredient with one or more solubilizers; and incorporating said solubilized active agent in a gastroretentive matrix having one or more swelling agents and one or more swelling enhancers.

29. The process according to claim 28 wherein the solubilization is done with melt granulation.

## Patentansprüche

1. Orale pharmazeutische Zusammensetzung zur gesteuerten Freisetzung, umfassend:
a. therapeutisch wirksame Menge eines oder mehrerer pharmakologisch aktiven Mittel(s), das/die geringe Bioverfügbarkeit zeigt;
b. ein oder mehrere Lösungsvermittler, wobei das Verhältnis von Arzneimittel zu Lösungsvermittler 20:1 bis 1:20 ist.
c. ein oder mehrere biokompatible(s) Quellmittel; und
d. ein Quellverstärker in einer Menge von 5 bis 90 Gewichtsprozent.
wobei das Quellmittel in Kombination mit dem Quellverstärker in Anwesenheit von Magenflüssigkeit aufquillt, sodass die Größe der Darreichungsform ausreichend angestiegen ist, um das Zurückhalten der Darreichungsform im Magen des Patienten bereitzustellen und graduell innerhalb des Gastrointestinaltraktes über einen langen Zeitraum erodiert.

2. Orale pharmazeutische Zusammensetzung zur gesteuerten Freisetzung nach Anspruch 1, wobei das pharmakologische Mittel ausgewählt ist aus der Gruppe bestehend aus: Anti-Ulcus-, antidiabetischen, Antikoagulations-, antithrombotischen, hypolipämischen, antiarrhythmischen, vasodilatorischen, antianginösen, antihypertensiven, vasoprotektiven Mitteln, Fertilitätsverstärkern, Wehenförderern und -hemmern, Kontrazeptivum, Antibiotikum, antimykotischen, antiviralen, Antikrebs-, entzündungshemmenden Mitteln, Analgetikum, Antiepileptikum, Anti-Parkinson-, Neuroleptikum, Hypnotikum, anxiolytischen, psychostimulierenden Mitteln, Antimigränemitteln, Antidepressivum, Antitussivum, Antihistaminikum oder Antiallergika.

3. Orale pharmazeutische Zusammensetzung zur gesteuerten Freisetzung nach Anspruch 1, wobei das pharmakologische Mittel ausgewählt ist aus der Gruppe bestehend aus Pentoxifyllin, Prazosin, Acyclovir, Levodopa, Nifedipin, Diltiazem, Naproxen, Flurbiprofen, Ketoprofen, Fenoprofen, Fentiazac, Oestradiolvalerat, Metoprolol, Sulpirid, Captopril, Cimetidin, Zidovudin, Nicardipin, Terfenadin, Salbutamol, Carbamazepin, Ranitidin, Enalapril, Simvastatin, Fluoxetin, Famotidin, Ganciclovir, Famiciclovir, Ciprofloxacin, Pentazocin, Omeprazol, Saquinavir, Ritonavir, Indinavir, Nelfinavir, Thiamphenicol, Calciumcarbonat, Clarithromycin, Azithromycin, Ceftazidim, Cyclosporin, Digoxin, Paclitaxel, Eisensalzen, Topiramat und Ketoconazol und Mischungen davon.

4. Orale pharmazeutische Zusammensetzung zur gesteuerten Freisetzung nach Anspruch 1, wobei das pharmakologische Mittel Acyclovir ist.

5. Orale pharmazeutische Zusammensetzung zur gesteuerten Freisetzung nach Anspruch 1, wobei der Lösungsvermittler ausgewählt ist aus der Gruppe bestehend aus hydrophilen Tensiden, lipophililen Tensiden und Mischungen davon.

6. Orale pharmazeutische Zusammensetzung zur gesteuerten Freisetzung nach Anspruch 1, wobei der Lösungsvermittler ausgewählt ist aus anionischen, nichtionischen, kationischen und zwitterionischen Tensiden.

7. Orale pharmazeutische Zusammensetzung zur gesteuerten Freisetzung nach Anspruch 1, wobei der Lösungsvermittler ein oder mehrere hydrophile(s) nichtionische(s) Tensid(e) umfasst, ausgewählt aus der Gruppe bestehend aus Polyethylenglycolfettsäureestern und hydrophilen Umesterungsprodukten eines Polyols mit zumindest einem Mitglied der Gruppe bestehend aus Triglyceriden, pflanzlichen Ölen und hydrierten pflanzlichen Ölen.

8. Orale pharmazeutische Zusammensetzung zur gesteuerten Freisetzung nach Anspruch 1, wobei der Lösungsvermittler ausgewählt ist aus PEG-20-Glycerylstearat, PEG-40 hydriertem Rizinusöl, PEG 6 Maisöl, Lauryl-Macrogol - 32 Glycerid, Stearoyl-Macrogol-Glycerid, Polyglyceryl-10-Monodioleat, Propylenglycololeat, Propylenglycoldioctanoat, Propylenglycolcaprylat/caprat, Glycerylmonooleat, Glycerolmonolinoleat, Glycerolmonostearat, PEG- 20 sorbitanmonolaurat, PEG - 4 Laurylether, Sacharosedistearat, Sacharosemonopalmitat, Polyoxyethylen-Polyoxypropylen-Block-Copolymer, Polyethylenglycol 660-hydroxystearat, Natriumlaurylsulfat, Natriumdodecylsulfat, Propylenglycolalginat, Natriumtaurocholat, Natriumglycocholat, Natriumdeoxycholat, Betainen, Polyethylenglycol und Mischungen davon.

9. Orale pharmazeutische Zusammensetzung zur gesteuerten Freisetzung nach Anspruch 1, wobei der Lösungsvermittler bevorzugt eine genau definierte Mischung aus Mono-, Di- und Triglyceriden und Mono- und Difettsäureestern von Polyethylenglycol ist.

10. Orale pharmazeutische Zusammensetzung zur gesteuerten Freisetzung nach Anspruch 1, wobei das Verhältnis von Arzneimittel zu Lösungsvermittler 10:1 bis 1:10 ist.

11. Orale pharmazeutische Zusammensetzung zur gesteuerten Freisetzung nach Anspruch 1, wobei das Verhältnis von Arzneimittel zu Lösungsvermittler 5:1 bis 1:5 ist.

12. Orale pharmazeutische Zusammensetzung zur gesteuerten Freisetzung nach Anspruch 1, wobei das biokompatible Quellmittel ausgewählt ist aus der Gruppe bestehend aus: Polyalkylenoxiden; Cellulosepolymeren; Acrylsäure- und Methacrylsäurepolymeren und Ester davon, Maleinsäureanhydrid-Polymeren; Polymaleinsäure, Poly(acrylamiden), Poly(olefinischen Alkohol)en; Poly(N-vinyllactamen); Polyolen; polyoxyethylierten Sacchariden; Polyoxazolinen; Polyvinylaminen; Polyvinylacetaten; Polyiminen; Stärke und Polymeren auf Stärkebasis; Polyurethanhydrogelen; Chitosan; Polysaccharidgummis; Zeinen; Polymeren auf Schellackbasis; und Copolymeren und Mischungen davon.

13. Orale pharmazeutische Zusammensetzung zur gesteuerten Freisetzung nach Anspruch 1, wobei das biokompatible Quellmittel ein oder mehrere hydrophile(s) Polymer(e) ist, ausgewählt aus der Gruppe bestehend aus Polyethylenoxid, Hydroxypropylcellulose, Hydroxypropylmethylcellulose, Hydroxyethylcellulose, Natriumcarboxymethylcellulose, Calciumcarboxymethylcellulose, Methylcellulose, Polyacrylsäure, Maltodextrin, vorgelatinisierte Stärke, Polyvinylalkohol und Mischungen davon.

14. Orale pharmazeutische Zusammensetzung zur gesteuerten Freisetzung nach Anspruch 1, wobei das Polyalkylenoxid ein oder mehrere hydrophile(s) Polymer(e) ist, ausgewählt aus der Gruppe bestehend aus Poly(ethylenoxid), Poly(ethylenoxide-co- propylenoxid), und Mischungen davon.

15. Orale pharmazeutische Zusammensetzung zur gesteuerten Freisetzung nach Anspruch 14, wobei das hydrophile Polymer Poly(ethylenoxid) ist.

16. Orale pharmazeutische Zusammensetzung zur gesteuerten Freisetzung nach Anspruch 1, wobei das biokompatible Quellmittel ein oder mehrere hydrophile(s) Polymer(e) ist und der Gehalt des hydrophilen Polymers in der Polymermatrix 5 bis 90 Gewichtsprozent beträgt.

17. Orale pharmazeutische Zusammensetzung zur gesteuerten Freisetzung nach Anspruch 16, wobei der Gehalt des hydrophilen Polymers in der Polymermatrix 10 bis 70 Gewichtsprozent beträgt.

18. Orale pharmazeutische Zusammensetzung zur gesteuerten Freisetzung nach Anspruch 17, wobei der Gehalt des hydrophilen Polymers in der Polymermatrix 15 bis 50 Gewichtsprozent beträgt.

19. Orale pharmazeutische Zusammensetzung zur gesteuerten Freisetzung nach Anspruch 1, wobei der Quellverstärker ausgewählt ist aus der Gruppe bestehend aus niedrig substituierter Hydroxypropylcellulose, mikrokristalliner Cellulose, vernetzter Natrium- oder Calciumcarboxymethylcellulose, Cellulosefaser, vernetztem Polyvinylpyrrolidon, vernetzter Polyacrylsäure, vernetztem Amberlit-Harz, Alginaten, kolloidalem Magnesiumaluminiumsilikat, Maisstärkegranulat, Reisstärkegranulat, Kartoffelstärkegranulat, vorgelatinisierter Stärke, Natriumcarboxymethylstärke und Mischungen davon.

20. Orale pharmazeutische Zusammensetzung zur gesteuerten Freisetzung nach Anspruch 1, wobei der Quellverstärker ausgewählt ist aus der Gruppe bestehend aus vernetzter Natrium-, Calciumcarboxymethylcellulose, vernetztem Polyvinylpyrrolidon, Natriumcarboxymethylstärke, vorgelatinierter Stärke und Mischungen davon.

21. Orale pharmazeutische Zusammensetzung zur gesteuerten Freisetzung nach Anspruch 1, wobei der Quellverstärker ein vernetztes Polyvinylpyrrolidon ist.

22. Orale pharmazeutische Zusammensetzung zur gesteuerten Freisetzung nach Anspruch 1, wobei der Gehalt des Quellverstärkers 10 bis 70 Gewichtsprozent beträgt.

23. Orale pharmazeutische Zusammensetzung zur gesteuerten Freisetzung nach Anspruch 1, wobei der Gehalt des Quellverstärkers 15 bis 50 Gewichtsprozent beträgt.

24. Orale pharmazeutische Zusammensetzung zur gesteuerten Freisetzung nach Anspruch 1, wobei die Zusammensetzung in der Form eines erweiterten Mehrschichtsystems ist, das eine erste Schicht, die zumindest einen pharmazeutischen Wirkstoff mit einer sofortigen Freisetzungseigenschaft aufweist; und eine zweite Schicht mit einer verzögerten Freisetzungseigenschaft umfasst, die zumindest einen pharmazeutischen Wirkstoff aufweist, der eine geringe Bioverfügbarkeit, einen oder mehrere Lösungsvermittler, ein oder mehrere biokompatible(s) Quellmittel und einen Quellverstärker zeigt.

25. Orale pharmazeutische Zusammensetzung zur gesteuerten Freisetzung nach Anspruch 24, wobei das Verhältnis des Wirkstoffs in der ersten Schicht zum Wirkstoff der zweiten Schicht in dem Bereich von 10:90 bis 90:10 bezogen auf das Gewicht liegt.

26. Orale pharmazeutische Zusammensetzung zur gesteuerten Freisetzung nach Anspruch 25, wobei die erste Schicht ferner ein Sprengmittel umfasst, ausgewählt aus der Gruppe bestehend aus Stärke, Natriumstärkegycolat, vorgelatinierter Stärke, vernetztem Polyvinylpyrrolidon, vernetzter Caboxymethylcellulose, Ionenausstauschharz und Mischungen davon.

27. Orale pharmazeutische Zusammensetzung zur gesteuerten Freisetzung nach Anspruch 26, wobei das Sprengmittel in einer Menge im Bereich von 0,25 % bis 10 %, weiter bevorzugt 0,5 % bis 5,0 % und insbesondere bevorzugt 1 % bezogen auf das Gewicht basierend auf dem Gesamtgewicht der Zusammensetzung vorliegt.

28. Verfahren zum Herstellen einer pharmazeutischen Zusammensetzung umfassend die Schritte des Lösens eines pharmazeutischen Wirkstoffs mit einem oder mehreren Lösungsvermittler(n); und Einbringen des löslichen Wirkstoffs in eine gastroretentive Matrix, die ein oder mehrere Quellmittel und ein oder mehrere Quellverstärker aufweist.

29. Verfahren nach Anspruch 28, wobei das Lösen mit Schmelzgranulation durchgeführt wird.

## Revendications

1. Composition pharmaceutique à libération contrôlée administrée par voie orale comprenant :
a. une quantité thérapeutiquement efficace d'un ou plusieurs agents pharmacologiquement actifs présentant une faible biodisponibilité ;
b. un ou plusieurs agents de solubilisation, le rapport du médicament sur ledit agent de solubilisation valant de 20:1 à 1:20 ;
c. un ou plusieurs agents gonflants biocompatibles ; et
d. un activateur de gonflement en une quantité de 5 à 90 % en poids ;
ledit agent gonflant en combinaison avec l'activateur de gonflement, gonflant en présence de fluide gastrique de sorte que la taille de la forme posologique soit suffisamment augmentée pour assurer la rétention de la forme posologique dans l'estomac d'un patient, et se dissolvant progressivement à l'intérieur du tractus gastrointestinal sur une durée prolongée.

2. Composition pharmaceutique à libération contrôlée administrée par voie orale selon la revendication 1, ledit agent pharmacologiquement actif étant choisi dans le groupe constitué par : les agents antiulcéreux, antidiabétiques, anticoagulants, antithrombiques, hypolipémiants, antiarythmiques, vasodilatateurs, antiangineux, antihypertenseurs, les agents vasoprotecteurs, les agents augmentant la fécondité, les agents stimulateurs et les agents inhibiteurs des contractions lors de l'accouchement, les agents contraceptifs, antibiotiques, antifongiques, antiviraux, anticancéreux, antiinflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques.

3. Composition pharmaceutique à libération contrôlée administrée par voie orale selon la revendication 1, ledit agent pharmacologiquement actif étant choisi dans le groupe constitué par la pentoxifylline, la prazosine, l'acyclovir, la lévodopa, la nifédipine, le diltiazem, le naproxène, le flurbiprofène, le kétoprofène, le fénoprofène, le fentiazac, le valérate d'oestradiol, le métoprolol, le sulpiride, le captopril, la cimétidine, la zidovudine, la nicardipine, la terfénadine, la salbutamol, la carbamazépine, la ranitidine, l'énalapril, la simvastatine, la fluoxétine, la famotidine, le ganciclovir, le famiciclovir, la ciprofloxacine, la pentazocine, l'oméprazole, le saquinavir, le ritonavir, l'indinavir, le nelfinavir, le thiamphénicol, le carbonate de calcium, la clarithromycine, l'azithromycine, la ceftazidime, la cyclosporine, la digoxine, le paclitaxel, les sels de fer, le topiramate et le kétoconazole et des mélanges de ceux-ci.

4. Composition pharmaceutique à libération contrôlée administrée par voie orale selon la revendication 1, ledit agent pharmacologiquement actif étant l'acyclovir.

5. Composition pharmaceutique à libération contrôlée administrée par voie orale selon la revendication 1, ledit agent de solubilisation étant choisi dans le groupe constitué par les tensioactifs hydrophiles, les tensioactifs lipophiles et des mélanges de ceux-ci.

6. Composition pharmaceutique à libération contrôlée administrée par voie orale selon la revendication 1, ledit agent de solubilisation étant choisi parmi les tensioactifs anioniques, non ioniques, cationiques et zwitterioniques.

7. Composition pharmaceutique à libération contrôlée administrée par voie orale selon la revendication 1, ledit agent de solubilisation comprenant un ou plusieurs tensioactifs hydrophiles non ioniques choisis dans le groupe constitué par les esters d'acides gras de polyéthylène glycol sorbitane et les produits de transestérifications hydrophiles d'un polyol avec au moins un élément du groupe constitué par les triglycérides, les huiles végétales et les huiles végétales hydrogénées.

8. Composition pharmaceutique à libération contrôlée administrée par voie orale selon la revendication 1, ledit agent de solubilisation étant choisi parmi le stéarate de PEG 20-glycéryle, l'huile de ricin hydrogénée de PEG 40, l'huile de maïs de PEG 6, le glycéride de macrogol 32 de lauryle, le glycéride de macrogol de stéaroyle, le monodioléate de polyglycéryle 10, l'oléate de propylène glycol, le dioctanoate de propylène glycol, le caprylate/caprate de propylène glycol, monooléate de glycéryle, le monolinoléate de glycérol, le monostéarate de glycérol, le monolaurate de PEG 20 sorbitane, l'éther laurylique de PEG 4, le distéarate de saccharose, le monopalmitate de saccharose, copolymère séquencé de polyoxyéthylène-polyoxypropylène, l'hydroxystéarate de polyéthylène glycol 660, le laurylsulfate de sodium, le dodécylsulfate de sodium, l'alginate de propylène glycol, le taurocholate de sodium, le glycocholate de sodium, le désoxycholate de sodium, les bétaïnes, le polyéthylène glycol et des mélanges de ceux-ci.

9. Composition pharmaceutique à libération contrôlée administrée par voie orale selon la revendication 1, ledit agent de solubilisation étant de préférence un mélange bien défini de mono-, di- et tri-glycérides et de mono- et di-esters d'acides gras de polyéthylène glycol.

10. Composition pharmaceutique à libération contrôlée administrée par voie orale selon la revendication 1, le rapport du médicament sur l'agent de solubilisation valant 10:1 à 1:10.

11. Composition pharmaceutique à libération contrôlée administrée par voie orale selon la revendication 1, ledit rapport du médicament sur l'agent de solubilisation valant 5:1 à 1:5.

12. Composition pharmaceutique à libération contrôlée administrée par voie orale selon la revendication 1, ledit agent gonflant biocompatible étant choisi dans le groupe constitué par : les oxydes de polyalkylènes ; les polymères cellulosiques ; les polymères d'acide acrylique et d'acide méthacrylique, et les esters de ceux-ci, les polymères d'anhydride maléique ; l'acide polymaléique, les polyacrylamides ; les alcools polyoléfiniques ; les poly(N-vinyl-lactames) ; les polyols ; les saccharides polyoxyéthylés ; les polyoxazolines ; les polyvinylamines ; les polyvinylacétates ; les polyimines ; l'amidon et les polymères à base d'amidon ; les hydrogels de polyuréthane ; le chitosane ; les gommes polysaccharidiques ; la zéine ; les polymères à base de gomme laque ; et des copolymères et mélanges de ceux-ci.

13. Composition pharmaceutique à libération contrôlée administrée par voie orale selon la revendication 1, ledit agent gonflant biocompatible étant un ou plusieurs polymères hydrophiles choisis dans le groupe constitué par l'oxyde de polyéthylène, l'hydroxypropylcellulose, l'hydroxypropylméthylcellulose, l'hydroxyéthylcellulose, la carboxyméthylcellulose de sodium, la carboxyméthylcellulose de calcium, la méthylcellulose, l'acide polyacrylique, la maltodextrine, l'amidon prégélatinisé, l'alcool polyvinylique et des mélanges de ceux-ci.

14. Composition pharmaceutique à libération contrôlée administrée par voie orale selon la revendication 1, ledit oxyde de polyalkylène étant un ou plusieurs polymères hydrophiles choisis dans le groupe constitué par l'oxyde de polyéthylène, le poly(oxyde d'éthylène-co-oxyde de propylène), et des mélanges de ceux-ci.

15. Composition pharmaceutique à libération contrôlée administrée par voie orale selon la revendication 14, ledit polymère hydrophile étant l'oxyde de polyéthylène.

16. Composition pharmaceutique à libération contrôlée administrée par voie orale selon la revendication 1, ledit agent gonflant biocompatible étant un ou plusieurs polymères hydrophiles et la teneur du polymère hydrophile dans la matrice polymère valant 5 à 90 pour cent en poids.

17. Composition pharmaceutique à libération contrôlée administrée par voie orale selon la revendication 16, ladite teneur du polymère hydrophile dans la matrice polymère valant 10 à 70 pour cent en poids.

18. Composition pharmaceutique à libération contrôlée administrée par voie orale selon la revendication 17, ladite teneur du polymère hydrophile dans la matrice polymère valant 15 à 50 pour cent en poids.

19. Composition pharmaceutique à libération contrôlée administrée par voie orale selon la revendication 1, ledit activateur de gonflement étant choisi dans le groupe constitué par une hydroxypropylcellulose faiblement substituée, une cellulose microcristalline, une carboxyméthylcellulose de sodium ou de calcium réticulée, une fibre de cellulose, une polyvinylpyrrolidone réticulée, un acide polyacrylique réticulé, une résine d'amberlite réticulée, des alginates, un silicate de magnésium-aluminium colloïdal, des granules d'amidon de maïs, des granules d'amidon de riz, des granules d'amidon de pomme de terre, un amidon prégélatinisé, le carboxyméthylamidon de sodium et des mélanges de ceux-ci.

20. Composition pharmaceutique à libération contrôlée administrée par voie orale selon la revendication 1, ledit activateur de gonflement étant choisi dans le groupe constitué par un sodium réticulé, la carboxyméthylcellulose de calcium, une polyvinylpyrrolidone réticulée, le carboxyméthylamidon de sodium, un amidon prégélatinisé et des mélanges de ceux-ci.

21. Composition pharmaceutique à libération contrôlée administrée par voie orale selon la revendication 1, ledit activateur de gonflement étant une polyvinylpyrrolidone réticulée.

22. Composition pharmaceutique à libération contrôlée administrée par voie orale selon la revendication 1, la teneur de l'activateur de gonflement valant 10 à 70 pour cent en poids.

23. Composition pharmaceutique à libération contrôlée administrée par voie orale selon la revendication 1, la teneur de l'activateur de gonflement valant 15 à 50 pour cent en poids.

24. Composition pharmaceutique à libération contrôlée administrée par voie orale selon la revendication 1, ladite composition étant sous la forme d'un système multicouche en expansion comprenant une première couche comportant au moins un ingrédient pharmaceutique actif dotée d'une propriété de libération immédiate ; et une seconde couche dotée d'une propriété de libération prolongée comportant au moins un ingrédient pharmaceutique actif présentant une faible biodisponibilité, un ou plusieurs agents de solubilisation, un ou plusieurs agents gonflants biocompatibles et un activateur de gonflement.

25. Composition pharmaceutique à libération contrôlée administrée par voie orale selon la revendication 24, le rapport dudit ingrédient actif dans ladite première couche sur ledit ingrédient actif dans ladite seconde couche se trouvant dans la plage de 10:90 à 90:10 en poids.

26. Composition pharmaceutique à libération contrôlée administrée par voie orale selon la revendication 25, ladite première couche comprenant en outre un agent de désintégration choisi dans le groupe constitué par l'amidon, le glycolate d'amidon de sodium, un amidon prégélatinisé, une polyvinylpyrrolidone réticulée, une carboxyméthylcellulose réticulée, une résine échangeuse d'ions et des mélanges de ceux-ci.

27. Composition pharmaceutique à libération contrôlée administrée par voie orale selon la revendication 26, ledit agent de désintégration étant présent en une quantité allant de 0,25 % à 10 %, plus préférablement de 0,5 à 5,0 % et idéalement de 1 % en poids par rapport au poids total de la composition.

28. Procédé de préparation d'une composition pharmaceutique comprenant les étapes de solubilisation d'un ingrédient pharmaceutique actif avec un ou plusieurs agents de solubilisation, et d'incorporation dudit agent actif solubilisé dans une matrice à rétention gastrique comportant un ou plusieurs agents gonflants et un ou plusieurs activateurs de gonflement.

29. Procédé selon la revendication 28, ladite solubilisation étant effectuée par granulation par fusion.
